# EUROPEAN PATENT APPLICATION

(11) **EP 2 689 802 A1**
(43) Date of publication of application: **29.01.2014**
(21) Application number: 12178114.0
(22) Date of filing: 26.07.2012
(51) Int. Cl.: A61P 15/04

(54) **Misoprostol formulation**

(71) Applicant: Ferring B.V., 2132 JX Hoofddorp (NL)
(72) Inventor: The designation of the inventor has not yet been filed
(74) Representative: Horner, Martin Grenville

(57) **Abstract**

A method of reducing the likelihood of infection requiring use of antibiotics during or after induction of labour in a female, comprises administering intravaginally to the female an insert comprising a cross-linked polyurethane reaction product of a polyethylene glycol, a triol and a diisocyanate, the insert containing 200 µg misoprostol;
the likelihood of infection being reduced in comparison to the administration of said insert containing 10 mg dinoprostone.

## Description

The present invention relates to the use of misoprostol for the induction of labour in a pregnant female, and in particular to the use of a sustained delivery device or insert containing substantially 200 µg misoprostol for intravaginal use. Such use includes methods of therapy and compositions for use in such methods.

Misoprostol is a synthetic analogue of prostaglandin E₁, and has been increasingly used for cervical ripening and labour induction administered both vaginally and orally. In some countries, it is is available as a 100 µg or 200 µg tablet, which is quartered or halved and then placed in the vagina every four to six hours. However, splitting tablets does not provide adequate control of dosing of misoprostol, nor is drug release from the tablet fragments steady or well defined.

Our patent application WO2004/029125 discloses a controlled release vaginal pessary comprising misoprostol in a cross-linked polyurethane polymer. Sustained release data in vitro is provided. Our patent application WO2006/013335 discloses that the long term storage properties of such misoprostol cross-linked polyurethane sustained release devices may be improved by maintaining the water content at a low level.

A prostaglandin-containing vaginal pessary has been available for a number of years under the trade mark Propess/Cervidil. It contains 10mg of the PGE2 prostaglandin dinoprostone in a cross-linked polyurethane matrix for sustained vaginal release. The pessary is contained within a net bag and has a retrieval cord, allowing the pessary to be withdrawn once the desired dose has been administered or when the woman reaches an appropriate stage during labour.

Cross-linked polyurethane formulations containing a prostaglandin are also disclosed in US4931288.

Patents US6642278, US2004/044080 and WO2003/011301 disclose other background information.

The gestation period in a human female is around 40 weeks. Induction of labour may be considered if the pregnancy progresses beyond the 40 week term without the baby being born. Generally, induction is considered if the pregnancy goes beyond the 41st or 42nd week. Induction may also be considered for a variety of other medical reasons. The so called "Bishop Score" is a pre-labour scoring system to assist in deciding whether induction of labour will be required. The Bishop Score grades patients who would be most likely to achieve successful induction. The duration of labour is inversely correlated with the Bishop Score; a score that exceeds 8 describes a patient most likely to achieve a successful vaginal birth. Bishop Scores of less than 6 usually require that a cervical ripening method be used before other methods. The score involves five factors viz. cervical dilation, cervical effacement, cervical consistency, cervical position, and foetal station.

Induced labour tends to be more painful for the women and can lead to increased use of analgesics. It is also possible that induction may lead to an increased likelihood of caesarean section delivery for the baby. Medical reasons for the inducement of labour include hypertension or pre-eclampsia in the mother. However, induction may have adverse events, such as uterine tachysystole, foetal heart rate (FHR) irregularities, meconium in amniotic fluid, poor foetal condition (Apgar score), postpartum haemorrhage, chorioamnionitis and poor neonatal respiration.

Misoprostol controlled release pessaries have been investigated for possible clinical use and results are disclosed in a number of references including Powers et al. Journal of Clinical Pharmacology 2008, 48: 26-34, Ewert et al., Obstet Gynecol 2006; 108: 1130-7, Wing et al., J Reprod Med 2008; 53: 695-696, Castaneda et al. American Jn of Obstet Gyneco 2005; 193; 1071-5, Rayburn et al., J Soc Gynecol Investig 2006; 13: 112-7, Pevzner et al, Obstet Gynecol 2009; 114: 261-7, Wing Obster Gynecol 2008; 112: 801-12, Wing et al., Obstet Gynecol 2011; 117: 533-41. Results of clinical trials are also disclosed in our publication WO2011/156812, where the principal basis of comparison is absence of drug or escalating misoprostol dosage. Generally speaking, these studies show improved speed to vaginal delivery using misoprostol 200 µg pessaries without increased rate of caesarean delivery.

The present application is based on the discovery of further surprising benefits of misoprostol - containing controlled release vaginal pessaries.

The present invention provides in one aspect a method of reducing the time from start of active labour to delivery after induction of labour in a female, which comprises administering intravaginally to the female an insert comprising a cross-linked reaction production of a polyethylene glycol, a triol and a diisocyanate, the insert containing 200 µg misoprostol; the time being reduced in comparison to the administration of said insert containing 10 mg dinoprostone.

Another aspect provides a method of reducing the likelihood of infection requiring use of antibiotics during or after induction of labour in a female, which comprises administering intravaginally to the female an insert comprising a cross-linked polyurethane reaction product of a polyethylene glycol, a triol and a diisocyanate, the insert containing 200 µg misoprostol;
the likelihood of infection being reduced in comparison to the administration of said insert containing 10 mg dinoprostone.

A further aspect provides a method of reducing the time of drug dosing during induction of labour in a female, which comprising administering intravaginally to the female an insert comprising a cross-linked polyurethane reaction product of a polyethylene glycol, a triol and a diisocyanate, the insert containing 200 µg misoprostol; the time being reduced in comparison to the administration of said insert containing 10 mg disoprostone.

The invention also relates to the therapeutic use of the misoprostol-containing insert in any of these methods in a human female; or method of manufacture thereof; and to the use of the misoprostol-containing insert for the induction of labour in a female suffering from any of the clinical situations described herein (hypertension, preeclampsia, intrauterine growth restriction, membranes rupture etc.). Labour-associated adverse effects may be reduced.

The effect of the misoprostol-containing insert is compared to a dinoprostone-containing insert in the same cross-linked polyurethane. The term "insert" refers to the polyurethane hydrogel sustained delivery device, which may be loaded with drug (misoprostol; or dinoprostone for comparison). The term MVI 200 (or just MVI) refers to a formulated polyurethane insert containing 200 µg misoprostol. The term DVI refers to a formulated polyurethane insert containing 10 mg dinoprostone, which is used as the basis for comparison in the experimental data herein. The drug-containing insert may also be referred to as a pessary. In the experimental data herein the term "insert" is also used to include drug-loaded inserts.

The insert provides a sustained controlled delivery of misoprostol vaginally to the patient. Retrieval means may be provided for withdrawal of the drug-containing insert at a desired time according to clinical need.

The female may be parous or nulliparous.

Induction of labour may be needed in a number of clinical situations, including hypertension and pre-eclampsia, Induction is commonly due to the female being post-term (normally 40 weeks), for example in the range 40 to 41 weeks, or greater than or equal to 41 weeks. Induction may also be due to intrauterine growth restriction, or premature rupture of membranes.

Oxytocin may be provided to the female, especially for less than 8 hours during first hospitalization.

A variety of infections may arise which require use of antibiotics, including chorioamnionitis. Such infections may be injurious to the mother or the baby (neonate). Infection may need to be treated intrapartum, post-partum or neonatally. Chorioamnionitis is caused by a (bacterial) infection and results in inflammation of the amnion and/or chorion (the foetal membranes). Chorioamnionitis is known to prolong labour. The signs and/or symptoms of chorioamnionitis may include, for example, a fever (temperature > 37.5°C), uterine tenderness, purulent vaginal discharge and/or persistent maternal or fetal tachycardia. The antibiotic usage is the total antibiotics of all kinds administered to the female or neonate during such time period.

Delivery of the baby may be vaginally or by cesarean section. Vaginal delivery is either spontaneous or with instrumental assistance.

Misoprostol may act in cervical ripening and labour induction. It is surprising that duration of labour is reduced - even after the misoprostol-containing insert is removed from the female vagina.

The misoprostol-containing insert is administered by introduction into the female at a time determined by the clinician. The time from administration to active labour (as defined herein) is referred to as the "time to active labour". Once active labour is initiated, the time to delivery of the baby is referred to as "the time from active labour to delivery"; and this is the duration of active labour. The dosing period is the time from insertion of the drug-containing insert into the female to removal thereof.

Experimental clinical trial data will now be presented by way of example.

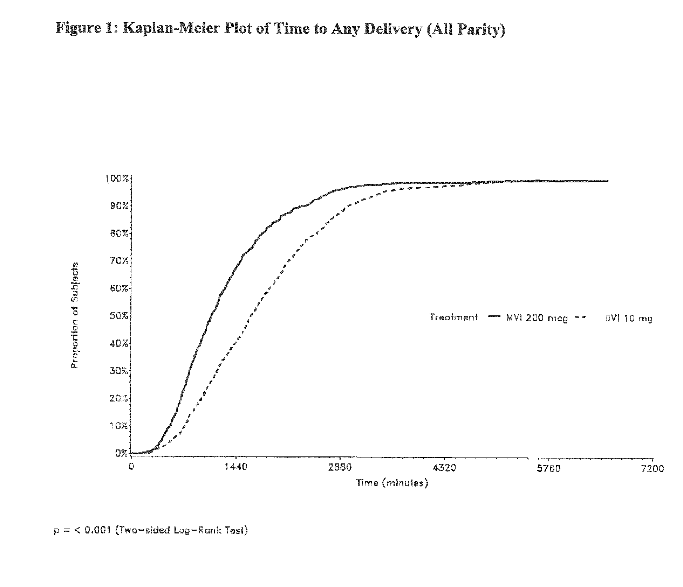

Figure 1 shows time to any delivery (including vaginal and cesarean delivery).

### Experimental

### Overall Study Design

This was a Phase III, double-blind, randomized, multicenter study of approximately 1,350 subjects at or near term gestation requiring cervical ripening and induction of labor.

Treatment consisted of administration of one randomly assigned MVI 200 or DVI. Nulliparous and parous subjects were randomized to their assigned treatments within their parity cohort in a double-blinded manner. The insert was to be kept in place for 24 hours unless events occurred that necessitated earlier removal (e.g., onset of active labor or intrapartum adverse event (AE)). Oxytocin was permitted after removal of the insert and completion of a 30-minute waiting period, if needed, to augment or induce labor. Enrollment was stratified by site and by parity, and randomization proceeded to that approximately 60% nulliparous subjects and 40% parous subjects were enrolled.

### Detailed Design

This Phase III study was a double-blind, randomized study comparing MVI 200 with DVI. (Cervidil^{®} [Forest Laboratories], Propess^{®} [Ferring Pharmaceuticals]) is an appropriate comparator for MVI 200 because it is the most commonly used marketed cervical ripening product available in the US and because it is identical in appearance to the MVI, allowing the study to be double-blinded. DVI is labeled in the US for a single administration of a single dose with removal at 12 hours. However, there is an adequate amount of drug in the reservoir to allow continuous dosing via controlled release for up to 24 hours. Because of this, the product is approved in some European countries for administration up to 24 hours. The FDA agreed to allow dosing of up to 24 hours for the DVI during this study in order to maintain the blinded nature of the study.

The study was randomized in order to prevent bias in the administration of different treatment groups and to attempt to have an even distribution of baseline characteristics across the arms of the study.

Eligible subjects were randomized to receive one of the following treatments: MVI 200 or DVI

Subjects were treated with one vaginal insert for up to 24 hours, one time only.

Intravenous oxytocin was permitted, when required, 30 minutes following removal of the study drug assuming no contraindications and active labor not present.

The MVI 200 and the DVI (Cervidil) were manufactured and released by Controlled Therapeutics (Scotland) Ltd.

The MVI had three components:
- a hydrogel polymer base measuring approximately 30 x 10 x 0.8 mm
- 200 mcg reservoir of misoprostol released at a controlled rate
- a retrieval tape consisting of inert woven polyester into which the polymer base was placed

The DVI had three components:
- a hydrogel polymer base measuring approximately 30 x 10 x 0.8 mm
- 10 mg reservoir of dinoprostone released at approximately 0.3 mg/hour
- a retrieval tape consisting of inert woven polyester into which the polymer base was placed.

Batch number information is provided in Table 1.

**Table 1: Investigational Drug (MVI and DVI) Batch Numbers**

| **Investigational Drug/Dose** | **Batch No.** | **Expiry Date** |
|---|---|---|
| MVI 200 mcg | MS10006 | 31 July 2013 |
| DVI 10 mg (Cervidil) | MA10K02/1 | 30 June 2013 |

For both the MVI and DVI, the polymer base was designed to absorb fluid from the vagina. As the polymer hydrates and swells, it creates a concentration gradient leading to a sustained release of misoprostol or dinoprostone for up to 24 hours. The polymer was a cross-linked polyurethane.

The MVI and DVI study drug inserts and packaging were identical in appearance (double-blinded). Each study drug kit consisted of a foil sachet with a preprinted subject number detailed on the label. The subject number differentiated study drug intended for nulliparous subjects from that intended for porous Subjects. A second self-adhesive label identical to that found on the study drug foil sachet was attached to the study drug foil label. The second self-adhesive label was placed on the study drug accountability form for that subject and kept with the study source documents.

The study drug kits were stored in a freezer. If unopened study drug was not used following removal from the freezer, it could have been returned to the freezer for use at a later date. The study drug could have been removed from and returned to the freezer multiple times as long as it was unopened and the total cumulative time outside the freezer was not more than 24 hours. Any study drug remaining out of the freezer for more than a total of 24 hours was discarded and its destruction documented.

### Selection and Timing of Dose for Each Patient

Subjects were randomized to receive one of the following in a double-blind manner: MVI 200 or DVI.

One randomized study drug was administered to each subject by the Investigator or qualified designee. The insert was placed high in the posterior vaginal fornix and positioned transversely. A minimal quantity of water-soluble lubricant could have been used to aid placement of the study drug. The insert was not pre-wetted or pre-swelled prior to insertion and obstetric cream was not used.

The subject remained in bed for at least 30 minutes after insertion to ensue that sufficient time was provided for the insert to hydrate and start to swell.

The subject was instructed to use caution when using the toilet or washing to avoid inadvertent removal of the insert.

Subjects were treated with study drug for up to 24 hours. The study drug was removed before 24 hours if there was clinical concern for the wellbeing of mother or baby or if an adverse event (AE) occurred:

If the study drug fell out of the vagina spontaneously or was mistakenly removed early, it was not replaced. At the time of removal, an obstetrician, midwife, obstetric nurse, or other qualified site staff removed the insert by gently pulling on the retrieval tape.

### Oxytocin Use

Oxytocin use was not permitted within 7 days prior to study drug administration and while the study drug was in situ.

Intravenous oxytocin was permitted, when required, 30 minutes following removal of the study drug, assuming no contraindications and no active labor. Earlier administration was permitted, if required, for treatment of an emergency situation.

### Onset of Active Labor and Delivery

The date and time of onset of active labor were to be recorded throughout the treatment period. Active labor was defined as progressive cervical dilatation to 4 cm with any frequency of contractions OR rhythmic, firm, adequate, quality uterine contractions causing progressive cervical change occurring at a frequency of three or more in 10 minutes and lasting 45 seconds or more.

At time of delivery of neonate, the following were recorded:
● Mode of delivery (spontaneous vaginal, instrumented vaginal, or cesarean)
   - If cesarean delivery, the reason for the cesarean delivery was recorded.
● Date and time of delivery of neonate.

### Adverse Events (AE)

An AE was defined as any untoward medical occurrence in a patient or clinical trial subject administered a medicinal product and that does not necessarily have a causal relationship with this treatment.

Subjects were questioned and observed for evidence of AEs, whether or not related to study drug.

Adverse events were collected through hospital discharge following delivery. Adverse events that occurred during the L&D period were categorized as intrapartum AEs. Following delivery, AEs were categorized as postpartum (maternal) or neonatal events.

### Summaries of Adverse Event Incidence Rates

Averse events were summarized by system organ class and preferred term for intrapartum, postpartum, and neonate events without regard to causality or relationship to study drug.

### Summary of Outcomes and Adverse Events of Special Interest

Safety assessments were also summarized for Outcomes and AEs of Special Interest. Treatment groups were compared using Fisher's exact tests for each of these outcomes or events. However, there was no correction for multiplicity; therefore, p-values should be interpreted with caution.

### Results

### Time to Any Delivery (Vaginal or Cesarean) During the First Hospitalization

Time to any delivery mode (vaginal or cesarean) was significantly shorter in MVI 200 subjects (Kaplan Meier median 1096.50 minutes [18.3 hours]) compared with DVI subjects (Kaplan Meier median 1639.50 minutes [27.3 hours]) (p<0,001). Time to any delivery was also significantly shorter in MVI 200 subjects compared with DVI subjects among both nulliparous subjects (p<0.001) and porous subjects (p<0.001). Kaplan Meier estimates of time to any delivery are presented Table 21.

Figure 1 shows time to any delivery for MVI200 versus DVI.

**Table 2: Kaplan-Meier Estimates of Time to Any Delivery**

| **Time From Study Drug Administration to Any Delivery (minutes)** | | **MVI 200 (N=678)** | **DVI (N=680)** |
|---|---|---|---|
| Any parity | | | |
| | N | 678 | 680 |
| | Median | 1096.50 | 1639.50 |
| | 95% Cl¹ | (1031.00, 1170.00) | (1573.00, 1731.00) |
| | p-value¹ | <0.001 | |
| | Number (%) of censored subjects² | 5(0.7) | 9(1.3) |
| | Discharged prior to delivery (imputed value: 4571 minutes) | 5(0.7) | 9(1.3) |

| Nulliparous subjects | | | |
|---|---|---|---|
| | N | 441 | 451 |
| | Median | 1304.0 | 1882.00 |
| | 95% Cl¹ | (1203.00, 1376.00) | (1764.00, 2016.00) |
| | p-value¹ | <0.001 | |
| | Number (%) of censored subjects² | 4(0.9) | 8(1.8) |
| | Discharged prior to delivery (imputed value: 4571 minutes) | 4 (0.9) | 8(1.8) |

| Parous subjects | | | |
|---|---|---|---|
| | N | 237 | 229 |
| | Median | 777.00 | 1155.00 |
| | 95% Cl¹ | (732.00, 823.00) | (1040.00, 1321.00) |
| | p-value¹ | <0.001 | |
| | Number (%) of censored subjects² | 1(0.4) | 1(0.4) |
| | Discharged prior to delivery (imputed value: 1642minutes) | 1 (0.4) | 1(0.4) |

| | | | |
|---|---|---|---|
| ¹Two-sided p-values and Cls were obtained from a Log-Rank Test. ²Subjects who did not deliver during their first hospitalization were censored using the longest time interval from study drug administration to L&D discharge, independent of treatment group. The Kaplan-Meier plot of time to any delivery the first hospitalization is presented in Figure 1 (all parity). | | | |

### Time to Active Labor During the First Hospitalization

Active labor was defined as progressive cervical dilatation to 4 cm with any frequency of contractions OR rhythmic, firm, adequate, quality uterine contractions causing progressive cervical change occurring at a frequency of three or more in 10 minutes and lasting 45 seconds or more.

Time to active labor was significantly shorter in MVI 200 subjects (median 726.50 minutes [12.1 hours]) compared to DVI subjects (median 1116.50 minutes [18.6 hours]) (p<0.001). Time to active labor was also significantly shorter in MVI 200 subjects compared with DVI subjects among both nulliparous subjects (p<0.001) and parous subjects (p<0.001). Kaplan-Meier estimates of time to active labor are presented in Table 3.

**Table3: Kaplan-Meier Estimates of Time to Active Labor**

| **Time From Study Drug Administration to Active Labor (minutes)** | **MVI 200 (N=678)** | **DVI (N=680)** |
|---|---|---|
| Any parity | | |
| N | 678 | 680 |
| Median | 726.50 | 1116.50 |
| 95% Cl¹ | (719.00,773.00) | (1083.00,1352.00) |
| p-value¹ | <0.001 | |
| Number (%) of censored subjects² | 47 (6.9) | 54 (7.9) |
| Discharged without active labor (imputed value: 5618 minutes) | 42 (6.2) | 45 (6.6) |
| Discharged prior to active labor (imputed value: 4571 minutes) | 5 (0.7) | 9 (1.3) |
| Nulliparous subjects | | |
| N | 441 | 451 |
| Median | 885.00 | 1444.0 |
| 95% Cl¹ | (810.00, 948.00) | (1352.00, 1558.00) |
| p-value¹ | <0.001 | |
| Number (%) of censored subjects² | 41 (9.3) | 50 (11.1) |
| Discharged without active labor (imputed value: 5618 minutes) | 37 (8.4) | 42 (9.3) |
| Discharged prior to active labor (imputed value: 4571 minutes) | 4 (0.9) | 8 (1.8) |
| Parous subjects | | |
| N | 237 | 229 |
| Median | 579.00 | 780.00 |
| 95% Cl¹ | (535.00, 616.00) | (715.00, 913.00) |
| p-value¹ | <0.001 | |
| Number (%) of censored subjects² | 6 (2.5) | 4 (1.7) |
| Discharged without active labor (imputed value: 1451 minutes) | 5 (2.1) | 3 (1.3) |
| Discharged prior to active labor (imputed value: 1642 minutes) | 1 (0.4) | 1 (0.4) |

| | | |
|---|---|---|
| ¹Two-sided p-values and CIs were obtained from a Log-Rank Test. ² Subjects who did not go into active labor during the first hospitalization were censored using the longest time interval from study drug administration to delivery during the first hospitalization, independent of treatment group. Subjects who, in their first hospitalization, were discharged prior to delivery or withdrew consent prior to delivery were censored using the longest time interval from study drug administration to L&D discharge, independent of treatment group. | | |

### Incidence of Cervical Ripening Success at 12 Hours

A higher percentage of subjects in the MVI 200 treatment group achieved the composite endpoint for cervical ripening at 12 hours than in the DVI treatment group (83.6% vs. 67.5%, p<0.001; Table 4). The treatment group difference was also statistically significant among both nulliparous subjects (p<0.001) and parous subjects (p<0.001).

**Table 4: Composite Endpoint for Cervical Ripening at 12 Hours**

| | | **MVI 200** | **DVI** |
|---|---|---|---|
| | | **(N=678)** | **(N=680)** |
| Any parity | | 678 | 680 |
| | Achieved composite endpoint for cervical ripening, n (%)¹ | 567 (83.6) | 459 (67.5) |
| | 95% CI² | (80.62%,86.34%) | (63.84%, 71.01%) |
| | p-valua³ | <0.001 | |
| | Nulliparous | 441 | 451 |
| | Achieved composite endpoint for cervical ripening, n (%) | 359(81.4) | 295 (65.4) |
| | 95% CI² | (77.45%, 84.93%) | (60.82%, 69.80%) |
| | p-value³ | <0.001 | |
| Parous | | 237 | 229 |
| | Achieved composite endpoint for cervical ripening, n (%)¹ | 208 (87.8) | 164 (71.6) |
| | 95% CI² | (82.90%, 91.65%) | (65.30%, 77.36%) |
| | p-value³ | <0.001 | |

| | | | |
|---|---|---|---|
| ¹ Achieved one or more of the following by 12 hours of treatment: increase from baseline in mBS ≥3, achievement of mBS ≥6, or vaginal delivery. If the subject had a cesarean delivery prior to hour 12, or if the subject had not delivered by hour 12 and the score was missing, the hour 6 Bishop score was used (LOCF). ² 95% exact binomial CI. ³ Two-sided p-values were obtained from Fisher's exact tests. | | | |

### Time from Active Labour to Delivery

Time from onset of active labour to delivery (the duration of labour) is given in Tables 5.1 to 5.3 for any delivery, vaginal delivery and cesarean delivery.

**Table 5.1**

| **Time from Active Labor to Delivery During First Hospitalization** | | | | |
|---|---|---|---|---|
| | | MVI 100 (N=678) | DVI (N=680) | Total (N=1358) |
| | | | | |
| Subjects with No Time to Active Labor | No Delivery During First Hospitalization | 5 | 9 | 14 |
| | Delivery but No Active Labor | 42 | 45 | 87 |
| | | | | |
| Time from Active Labor to Delivery During First (min) | N | 631 | 626 | 1257 |
| | Mean | 380.8 | 487.1 | 433.8 |
| | SD | 354.42 | 365.83 | 363.91 |
| | Median | 285.0 | 414.5 | 374.0 |
| | Min, Max | 0, 3266 | 0,3309 | 0,3309 |
| | | | | |

**Table 5.2**

| **Time from Active Labor to Vaginal Delivery During First Hospitalization** | | | | |
|---|---|---|---|---|
| | | **MVI 100 (N=678)** | **DVI (N=680)** | **Total (N=1358)** |
| | | | | |
| Subjects with No Time to Active Labor | No Vaginal Delivery During First Hospitalization | 181 | 193 | 374 |
| | | | | |
| Time from Active Labor to Vaginal Delivery During First Hospitalisation (min) | N | 497 | 487 | 984 |
| | Mean | 326.1 | 432.3 | 378.6 |
| | SD | 271.51 | 306.94 | 295.20 |
| | Median | 257.0 | 378.0 | 316.0 |
| | Min, Max | 0, 1874 | 0,2266 | 0,2266 |
| | | | | |

**Table 5.3**

| **Time from Active Labor to Cesarean Delivery During First Hospitalization** | | | | |
|---|---|---|---|---|
| | | **MVI 100 (N=678)** | **DVI (N=680)** | **Total (N=1358)** |
| | | | | |
| Subjects with No Time to Active Labor | No Cesarean Delivery During First Hospitalization | 502 | 496 | 998 |
| | Cesarean Delivery but No Active Labor | 42 | 45 | 87 |
| | | | | |
| Time from Active Labor to Cesarean Delivery During First Hospitalization (min) | N | 134 | 139 | 273 |
| | Mean | 583.8 | 679.4 | 632.5 |
| | SD | 513.16 | 475.88 | 495.93 |
| | Median | 460.5 | 585.0 | 545.0 |
| | Min, Max | 13,3266 | 0,3309 | 0,3309 |
| | | | | |

### Incidence of Subject/Neonate Antibiotic Use During First Hospitalization

Overall, systemic antibiotic use was lower in the MVI 200 treatment group compared with the DVI treatment group.

The percentage of subjects who received intrapartum concomitant antibiotics (e.g., antibacterials for systemic use) was 8.1% in the MVI 200 treatment group and 11.3% in the DVI treatment group. Intrapartum concomitant antibiotics received by ≥1.0% of subjects overall included ampicillin (4.0% MVI 200, 6.2% DVI), gentamicin (3.7% MVI 200, 6.2% DVI), clindamycin (1.0% MVI 200, 1.9% DVI), and nitrofurantoin (1.2% MVI 200, 1.0% DVI).

The percentage of subjects who received postpartum concomitant antibiotics (e.g., antibacterials for systemic use) was 5.6% in the MVI 200 treatment group and 9.6% in the DVI treatment group. Postpartum concomitant antibiotics received by ≥1.0% of subjects overall included gentamicin (3.7% MVI 200, 5.9% DVI), ampicillin (2.7% MVI 200, 5.0% DVI), and clindamycin (2.5% MVI 200, 3.8% DVI).

The percentage of subjects who received neonate concomitant antibiotics (e.g., antibacterials for systemic use) was 7.2% in the MVI 200 treatment group and 9.7% in the DVI treatment group. Neonate concomitant antibiotics received by ≥1.0% of subjects overall included ampicillin (7.1% MVI 200, 9.4% DVI) and gentamicin (6.5% MVI 200, 9.0% DVI).

Evidence of reduced total antibiotic use in various scenarios is provided in the following Tables. The scenarios are in respect of parous or nuiliparous females, induction for hypertension, pre-eclampsia, post-term (40 - 41 weeks, greater than or equal to 41 weeks), intrauterine growth restriction, premature rupture of membranes, oxytocin use, and vaginal or cesarean delivery.

**Table 6 (Reduced Antibiotics Usage)**

| **Nulliparous Subjects** | | | |
|---|---|---|---|
| | **MVI 200 (N=441)** | **DVI (N=451)** | **Total (N=892)** |
| Neonatal IV/IM Antibiotic Use | 39 (8.8%) | 55 (12.2%) | 94 (10.5% |
| Neonatal IV/IM Antibiotic Use | 35 (7.9%) | 53 (11.8%) | 88 (9.9%) |
| Postpartum IV/IM Antibiotic Use | 29 (6.6%) | 48 (10.6%) | 77 (8.6%) |
| Chorioamnionitis | 34 (7.7%) | 53 (11.8%) | 87 (9.8%) |

**Table 7 (Reduced Antibiotics Usage)**

| **Parous Subjects** | | | |
|---|---|---|---|
| | **MVI 200 (N=237)** | **DVI (N=229)** | **Total (N=466)** |
| Neonatal IV/IM Antibiotic Use | 8 (3.4%) | 11 (4.8%) | 19 (4.1%) |
| Intrapartum IV/IM Antibiotic Use | 3 (1.3%) | 6 (2.6%) | 9 (1.9%) |
| Postpartum IV/IM Antibiotic Use | 2 (0.8%) | 9 (3.9%) | 11 (2.4%) |
| Chorioamnionitis | 4 (1.7%) | 6 (2.6%) | 10 (2.1%) |

**Table 8 (Reduced Antibiotics Usage)**

| **Subjects Inducted for Hypertension** | | | |
|---|---|---|---|
| | **MVI 200 (N=79)** | **DVI (N=86)** | **Total (N=165)** |
| Neonatal IV/IM Antibiotic Use | 3 (3.8%) | 9 (10.5%) | 12 (7.3%) |
| Intrapartum IV/IM Antibiotic Use | 3 (3.8%) | 9 (10.5%) | 12 (7.3%) |
| Postpartum IV/IM Antibiotic Use | 2 (2.5%) | 10 (1.6%) | 12 (7.3%) |
| Chorioamnionitis | 1 (1.3%) | 8 (9.3%) | 9 (5.5%) |

**Table 9 (Reduced Antibiotics Usage)**

| **Subjects Inducted for Hypertension** | | | |
|---|---|---|---|
| | **MVI 200 (N=71)** | **DVI (N=59)** | **Total (N=130)** |
| Neonatal IV/IM Antibiotic Use | 4 (5.6%) | 4 (6.8%) | 8 (6.2%) |
| Intrapartum IV/IM Antibiotic Use | 4 (5.6%) | 5 (8.5%) | 9 (6.9%) |
| Postpartum IV/IM Antibiotic Use | 7 (9.9%) | 4 (6.8%) | 11 (8.5%) |
| Chorioamnionitis | 4 (5.6%) | 4 (6.8%) | 8 (6.2%) |

**Table 10.1 (Reduced Antibiotics Usage)**

| **Subjects Inducted for Post Term** | | | |
|---|---|---|---|
| | **MVI 200 (N=210)** | **DVI (N=227)** | **Total (N=437)** |
| Neonatal IV/IM Antibiotic Use | 15 (7.1%) | 32 (14.1%) | 47 (10.8%) |
| lntrapartum IV/IM Antibiotic Use | 14 (6.7%) | 26 (11.5%) | 40 (9.2%) |
| Postpartum IV/IM Antibiotic Use | 11 (5.2%) | 28 (12.3%) | 39 (8.9%) |
| Chorioamnionitis | 17 (8.1%) | 32 (14.1%) | 49 (11.2%) |

**Table10.2 (Reduced Antibiotics Usage)**

| **Subjects Inducted for Post-Term (40 - 41 weeks)** | | | |
|---|---|---|---|
| | **MVI 200 (N=91)** | **DVI (N=115)** | **Total (N=206)** |
| Neonatal Antibiotic Use | 7 (7.7%) | 17 (14.8%) | 24 (11.7%) |
| Intrapartum IV/IM Antibiotic Use | 8 (8.8%) | 12 (10.4%) | 20 (9.7%) |
| Postpartum IV/IM Antibiotic Use | 7 (7.7%) | 11 (9.6%) | 18 (8.7% |
| Chorioamnionitis | 10 (11.0%) | 15 (13.0%) | 25 (12.1%) |

**Table 10.3 (Reduced Antibiotics Usage)**

| **Subjects Inducted for Post Term (>= 41 weeks)** | | | |
|---|---|---|---|
| | **MVI 200 (N=119)** | **DVI (N=112)** | **Total (N=231)** |
| Neonatal IV/IM Antibiotic Use | 8 (6.7%) | 15(13.4%) | 23 (10.0%) |
| Intrapartum IV/IM Antibiotic Use | 6 (5.0%) | 14(12.5%) | 20 (8.7%) |
| Postpartum IV/IM Antibiotic Use | 4 (3.4%) | 17 (15.2%) | 21 (9.1%) |
| Chorioamnionitis | 7(5.9%) | 17(15.2%) | 24 (10.4%) |

**Table 11 (Reduced Antibiotics Usage)**

| **Subjects Inducted for Intrauterine Growth Restriction** | | | |
|---|---|---|---|
| | **MVI 200 (N=35)** | **DVI (N=35)** | **Total (M=70)** |
| Neonatal IV/IM Antibiotic Use | 0(0.0%) | **3** (8.6%) | 3 (4.3%) |
| Intrapartum IV/IM Antibiotic Use | 1 (2.9%) | 3 (8.6%) | 4 (5.7%) |
| Postpartum IV/IM Antibiotic Use | 0 (0.0%) | 1 (2.9%) | 1 (14%) |
| Chorioamnionitis | 1 (2.9%) | 1 (2.9%) | 2 (2.9%) |

**Table 12 (Reduced Antibiotics Usage)**

| **Subjects Inducted for Hypertension** | | | |
|---|---|---|---|
| | **MVI 200 (N=22)** | **DVI (N=25)** | **Total (N=47)** |
| Neonatal IV/IM Antibiotic Use | 3 (13.6%) | 5 (20.0%) | 8 (17.0%) |
| Intrapartum IV/IM Antibiotic Use | 3 (13.6%) | 4 (16.0%) | 7 (14.9%) |
| Postpartum IV/IM Antibiotic Use | 2 (9.1%) | 4(16.0%) | 6 (12.8%) |
| Chorioamnionitis | 2 (9.1%) | 4(16.0%) | 6 (12.8%) |

**Table 13.1 (Reduced Antibiotics Usage)**

| **Subjects with Oxytocin Duration <8 Hours in First Hospitalization)** | | | |
|---|---|---|---|
| | **MVI 200 (N=189)** | **DVI (N=218)** | **Total (N=407)** |
| Neonatal IV/IM Antibiotic Use | 12 (6.3%) | 16 (7.3%) | 28 (6.9%) |
| Intrapartum IV/IM Antibiotic Use | 7 (3.7%) | 14 (6.4%) | 21 (5.2%) |
| Postpartum IV/IM Antibiotic Use | 7 (3.7%) | 17 (7.8%) | 24 (5.9%) |
| Chorioamnionitis | 10 (5.3%) | 13 (6.0%) | 23 (5.7%) |

**Table 13.2 (Reduced Antibiotics Usage)**

| **Subjects with Oxytocin Duration >=8 Hours in First Hospitalization)** | | | |
|---|---|---|---|
| | **MVI 200 (N=132)** | **DVI (N=279)** | **Total (N=411)** |
| Neonatal IV/IM Antibiotic Use | 26 (19.7%) | 43 (15.4%) | 69 (16.8%) |
| Intrapartum IV/IM Antibiotic Use | 21 (15.9) | 39 (14.0%) | 60 (14.6%) |
| Postpartum IV/IM Antibiotic Use | 16 (12.1%) | 34(12.2%) | 50 (12.2%) |
| Chorioamnionitis | 22(16.7%) | 41(14.7%) | 63(15.3%) |

**Table 14.1 (Reduced Antibiotics Usage)**

| **Subjects with Vaginal Delivery During First Hospitalization)** | | | |
|---|---|---|---|
| | **MVI 200 (N=497)** | **DVI (N=487)** | **Total (N=984)** |
| Neonatal IV/IM Antibiotic Use | 23 (4.6%) | 38 (7.8%) | 61 (6.2%) |
| Intrapartum IV/IM Antibiotic Use | 19 (3.8%) | 27 (5.5%) | 46 (4.7%) |
| Postpartum IV/IM Antibiotic Use | 10 (2.0%) | 27 (5.5%) | 45 (4.6%) |
| Chorioamnionitis | 18 (3.6%) | 27 (5.5%) | 45 (4.6%) |

**Table 14.2 (Reduced Antibiotics Usage)**

| **Subjects with Cesarean Delivery During First Hospitalization)** | | | |
|---|---|---|---|
| | **MVI 200 (N=176)** | **DVI (N=184)** | **Total (N=360)** |
| Neonatal IV/IM Antibiotic Use | 24 (13.6%) | 28 (15.2%) | 52 (14.4%) |
| Intrapartum IV/IM Antibiotic Use | 19 (10.8%) | 32 (17.4%) | 51 (14.2%) |
| Postpartum IV/IM Antibiotic Use | 21 (11.9%) | 32 (17.4%) | 53 (14.7) |
| Chorioamnionitis | 20 (11.4%) | 31 (16.8%) | 51 (14.2%) |

### Extent of Exposure

The MVI is designed to be removed upon the occurrence of various clinical events (e.g., upon onset of active labor). Thus, variation in the duration of exposure (vaginal insert *in situ)* was expected, as each subject had study drug removed when exogenous prostaglandin was no longer desirable. Discontinuation of study drug prior to 24 hours was more often due to an efficacy reason such as onset of active labor (43.8% MVI 200, 34.1 % DVI) than an AE (11.4% MVI 200, 4.0% DVI). Of note, the percentage of subjects with study drug *in situ* for >20 hours was 16.3% in the MVI 200 treatment group and 41.1% in the DVI treatment group.

Time of study drug *in situ* was statistically significantly shorter in the MVI 200 treatment group than in the DVI treatment group (median: 712.6 minutes [11.9 hours] vs. 983.0 minutes [16.4 hours]). Duration of study drug *in situ* is summarized in Table 15.

**Table 15: Duration of Study Drug In situ**

| | | **MVI 200 (N=678)** | **DVI (N=680)** | **p-value¹** |
|---|---|---|---|---|
| Duration of study drug *in situ* (minutes) | | | | <0.001 |
| | N | 673 | 676 | |
| | Mean (SD) | 712.6 (391.52) | 983.0 (435.84) | |
| | Median | 616.0 | 1034.5 | |
| | Minimum, maximum | 60,1508 | 48, 1560 | |
| Exposure interval, n (%) | | | | |
| | ≤240 minutes (≤4 hours) | 38 (5.6) | 28 (4.1) | |
| | >240 to 480 minutes (>4 to 8 hours) | 193 (28.7) | 79 (11.7) | |
| | >480 to 720 minutes (>8 to 12 hours) | 182 (27.0) | 116 (17.2) | |
| | >720 to 960 minutes (>12 to 16 hours) | 96(14.3) | 94 (13.9) | |
| | >960 to 1200 minutes (>16 to 20 hours) | 54 (8.0) | 81 (12.0) | |
| | >1200 to 1440 minutes (>20 to 24 hours) | 51 (7.6) | 125 (18.5) | |
| | >1440 minutes (>24 hours) | 59 (8.8) | 153 (22.6) | |

| | | | | |
|---|---|---|---|---|
| ¹ Two-sided p-value was obtained from a one-way ANOVA model. | | | | |

### Outcomes and Treatment-Emergent Adverse Events of Special Interest

For ease of review, a summary of outcomes and AEs of interest in this obstetric setting across the three AE reporting periods of intrapartum, postpartum, and neonatal is provided in Table 16. This table also includes events that are not AEs but provide important safety information, such as Apgar scores, rate of cesarean delivery, intensive care unit (ICU) admission, and antibiotic use.

**Table 16: Summary of Outcomes and Treatment-Emergent Adverse Events of special Interest**

| | | **Number (%) of Subjects** | | | |
|---|---|---|---|---|---|
| | | **MVI 200 (N=678)** | **DVI (N=680)** | **p-value¹** | |
| Uterine tachysystole (AE) | | 90(13.3) | 27 (4.0) | <0.001 | |
| | Requiring treatment (without FHR involvement) | 25 (3.7) | 9 (1.3) | | |
| | With FHR involvement (late decelerations, bradycardia, or prolonged decelerations) | 70(10.3) | 18(2.6) | | |
| Uterine tachysystole (non-AE) | | 291 (42.9) | 150 (22.1) | <0.001 | |
| Category II FHR patterns (AE) | | 169 (24.9) | 175 (25.7) | 0.755 | |
| Category II FHR patterns (non-AE) | | 472 (69.6) | 447 (65.7) | 0.132 | |
| Category III FHR patterns | | 9 (1.3) | 5 (0.7) | 0.299 | |
| Intrapartum resuscitations | | 85 (12.5) | 66 (9.7) | 0.102 | |
| Tocolysis use | | 83(12.2) | 28 (4.1) | <0.001 | |
| Meconium in amniotic fluid | | 120 (17.7) | 92(13.5) | 0.036 | |
| Cesarean delivery during first hospitalization | | 176 (26.0) | 184(27.1) | 0.667 | |
| Instrumented vaginal delivery during first hospitalization | | 43 (6.3) | 35 (5.1) | 0.353 | |
| Minute 1 Apgar score low (<7) | | 80 (11.8) | 79 (11.6) | 0.933 | |
| Minute 6 Apgar score low (<7)² | | 14(2.1) | 7 (1.0) | 0.130 | |
| | Intrapartum fetal acidosis | 7 (1.0) | 3 (0.4) | 0.224 | |
| Neonatal encephalopathy | | 4 (0.6) | 1 (0.1) | 0.217 | |
| Neonatal ICU admissions | | 61 (9.0) | 71 (10.4) | 0.410 | |
| Intrapartum ICU admissions | | 1 (0.1) | 1 (0.1) | 1.000 | |
| Postpartum ICU admissions | | 0 | 0 | | |
| Postpartum hemorrhage | | 42 (6.2) | 40 (5.9) | 0.821 | |
| Neonatal intravenous/intramuscular antibiotic use | | 49 (7.2) | 66 (9.7) | 0.119 | |
| Intrapartum intravenous/intramuscular antibiotic use | | 55 (8.1) | 77 (11.3) | 0.054 | |
| Postpartum intravenous/intramuscular antibiotic use | | 38 (5.6) | 65 (9.6) | 0.007 | |

| | | | | | |
|---|---|---|---|---|---|
| ¹ Two-sided p-value was obtained from Fisher's exact tests. ² Based on the reported 5-minute Apgar score, which differs from the AE data because Subject 18038 reported a single AE of hypoxic-ischaemic encephalopathy based on a constellation of symptoms, including low 5-minute Apgar. | | | | | |

### Conclusions

- MVI 200 reduced time to vaginal delivery, time to any delivery, and time to onset of active labor compared with DVI.
- MVI 200 reduced pre-delivery oxytocin use compared with DVI.
- MVI 200 had a greater percentage of subjects with vaginal delivery within 12 and 24 hours, any delivery within 12 and 24 hours, and cervical ripening success at 12 hours compared with DVI.
- Results of pharmacoeconomic endpoints demonstrated decreases in duration in L&D, percentage of subjects requiring pre-delivery oxytocin, and duration of maternal hospitalization with MVI 200 compared with DVI.
- MVI200 reduced time of active labor compared with DVI.
- MVI200 reduced total antibiotic usage intrapartum, post-partum and neonatally compared with DVI.
- MVI200 reduced the drug dosing time compared with DVI.

The present invention relates to these new and surprising results, as set out in the claims.

## Claims

1. A method of reducing the likelihood of infection requiring use of antibiotics during or after induction of labour in a female, which comprises administering intravaginally to the female an insert comprising a cross-linked polyurethane reaction product of a polyethylene glycol, a triol and a diisocyanate, the insert containing 200 µg misoprostol;
the likelihood of infection being reduced in comparison to the administration of said insert containing 10 mg dinoprostone.

2. A method according to claim 1, wherein the female is nulliparous.

3. A method according to claim 1, wherein the female is parous.

4. A method according to claim 1, wherein induction is because of hypertension.

5. A method according to claim 1, wherein induction is because of preeclampsia.

6. A method according to claim 1, wherein induction is because the female is post-term.

7. A method according to claim 6, wherein the female is post-term in the range 40 to 41 weeks.

8. A method according to claim 6, wherein the female is post-term in the range greater than or equal to 41 weeks.

9. A method according to claim 1, wherein induction is due to intrauterine growth restriction.

10. A method according to claim 1, wherein induction is due to premature rupture of membranes.

11. A method according to claim 1, wherein the female is provided with oxytocin for less than 8 hours during first hospitalization.

12. A method according to claim 1, wherein the infection is chorioamnionitis.

13. A method according to any preceding claim, wherein delivery is vaginal.

14. A method according to any preceding claim, wherein delivery is cesarean.

15. A method according to any preceding claim, which is intrapartum.

16. A method according to any preceding claim, which is post-partum.

17. A method according to any preceding claim, which is neonatal.

18. A method of reducing the time from start of active labour to delivery after induction of labour in a female, which comprises administering intravaginally to the female an insert comprising a cross-linked polyurethane reaction product of a polyethylene glycol, a triol and a diisocyanate, the insert containing 200 µg misoprostol;
the time being reduced in comparison to the administration of said insert containing 10 mg dinoprostone.

19. A method according to claim 18, wherein delivery is vaginal.

20. A method according to claim 18, wherein delivery is

21. A method of reducing the time of drug dosing during induction of labour in a female, which comprises administering intravaginally to the female an insert comprising a cross-linked polyurethane reaction product of a polyethylene glycol, a triol and a diisocyanate, the insert containing 200 µg misoprostol;
the time being reduced in comparison to the administration of said insert containing 10 mg dinoprostone.

22. An insert comprising a cross-linked polyurethane reaction product of a polyethylene glycol, a triol and a diisocyanate and containing 200 µg misoprostol for use in the method of any preceding claim.
